# EUROPEAN PATENT SPECIFICATION

(11) **EP 2 632 463 B1**
(45) Date of publication and mention of the grant of the patent: **04.04.2018**
(21) Application number: 11836886.9
(22) Date of filing: 20.10.2011
(51) Int. Cl.: A61K 31/496, A61K 47/12, A61K 47/20, A61K 47/22, A61K 9/06, A61K 9/08, A61K 47/10, A61K 47/32, A61K 47/44, A61K 9/70

(54) **ARIPIPRAZOLE COMPOSITIONS AND METHODS FOR THEIR TRANSDERMAL DELIVERY**
ARIPIPRAZOLZUSAMMENSETZUNGEN UND VERFAHREN ZU IHRER TRANSDERMALEN VERABREICHUNG
COMPOSITIONS D'ARIPIPRAZOLE ET MÉTHODES POUR LEUR ADMINISTRATION TRANSDERMIQUE

(30) Priority: 28.10.2010 US 407591 P
(43) Date of publication of application: 04.09.2013
(73) Proprietor: Aequus Pharmaceuticals Inc., Vancouver, BC V6C 1S4 (CA)
(72) Inventor: PLAKOGIANNIS, Fotios, M., Whitestone New York 11357 (US); HOSSAIN, Muhammed, Anwar, Congers New York 10920 (US)
(74) Representative: HGF Limited
(86) International application number: PCT/US2011/057080
(87) International publication number: WO 2012/058091

(56) References cited:
- WO-A2-2007/035348
- US-A1- 2004 170 672
- US-A1- 2007 032 651
- US-A1- 2008 112 986
- US-A1- 2009 156 813
- US-A1- 2010 015 195

## Description

### Field of the Invention

The present invention relates to the field of transdermal delivery of pharmaceutical compositions, which have an acceptable in vitro performance and good bioavailability. In particular, the transdermal pharmaceutical compositions of the present invention include liquids or gels of aripiprazole in a patch dosage form.

### Background of Invention

Aripiprazole (ARPZ) is the first of a new class of atypical antipsychotics (third generation). Biochemically, ARPZ is a partial agonist of the D2 family of dopamine receptors.^{1,2} It is active against positive and negative symptoms of schizophrenia.^{3,4} ARPZ is a quinolinone derivative, white crystalline powder, practically insoluble in water, with a low melting point (135-140°C), MW 448,38g/mole and partition coefficient of 4.54.

The following prior art describes other examples of transdermal delivery systems of aripiprazole. US2004/170672 A1 describes transdermal patches comprising aripiprazole containing, for example, a permeation enhancer (e.g. Brij 30), propanediol and silicone adhesive. WO2007/035348 A2 describes pharmaceutical compositions comprising nanoparticulate aripiprazole and at least one surface stabilizer. The composition was formulated for a range of uses including topical administration. However, none of the prior art describes a solvent combination as disclosed in the present invention for providing good skin permeation.

### Description of the Invention

In order that the invention may be more fully understood it will now be described, by way of example only, and with reference to the accompanying drawings, in which:
Figure 1 shows the Effect of Drug Concentration on the Flux of ARPZ through Cellulose Membrane from 0.5% Carbopol 971 Gel Systems;
Figure 2 shows the Cumulative Amount of 5% ARPZ Permeated through Cadaver Skin from 0.5% Carbopol Gel System,
Figure 3 shows the Cumulative Amount of Drug Permeated through Human Cadaver Skin from 5% ARPZ in 0.5% Carbopol Gel Systems with Enhancers (Fatty Acids).

### Example

ARPZ is practically insoluble in water and has been formulated as a liquid and gel dosage form (Table 1). All reported values are in weight/volume percentage (W/V)

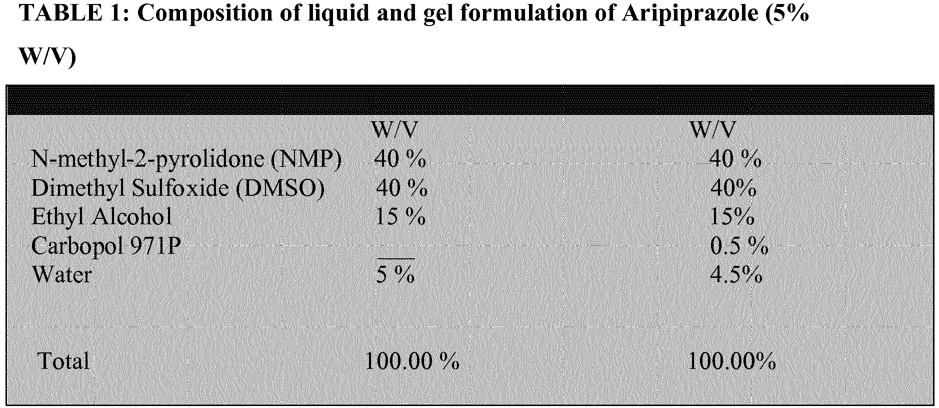

An optimal mixture design of experiments was used to select the levels of the formulation variables. The optimum composition of a 1% W/V to 20% W/V ARPZ liquid formulation was predicted to have NMP 40%, DMSO 40%, Alcohol 15% and water 5% (Table 1). The gel formulation should contain a gelling agent in the range of about 0.1% to 5% W/V and the optimum APRZ composition should range from about 1% W/V to 20% W/V with about .5% W/V of the gelling agent. Therefore, the gel formulation was predicted to have a NMP of 40%, DMSO 40%, Alcohol 15%, Carbopol 971 0.5%, and Water 4.5% (Table 1). However, Table 2 lists other combinations that also could produce successful liquid and gel ARPZ formulations.

**TABLE 2. Concentration Ranges of N-Methyl-2-Pyrolidone (NMP), Dimethl Sulfoxide (DMSO), Ethyl Alcohol, and Water in Liquid Aripiprazole Formulation. Formulation 4 describes the invention. Examples 1-3 and 5-12 in Table 2 do not fall under the scope of the invention.**

| **Formulation** | **NMP** | **DMSO** | **Alcohol** | **Water** |
|---|---|---|---|---|
| 1. | 50 | 50 | _ | ---- |
| 2. | 40 | 40 | 20 | ---- |
| 3. | 40 | 40 | ---- | 20 |
| 4. | 40 | 40 | 15 | 5 |
| 5. | 40 | 40 | 10 | 10 |
| 6. | 40 | 40 | 5 | 15 |
| 7. | 30 | 30 | 20 | 20 |
| 8. | 30 | 30 | 30 | 10 |
| 9. | 30 | 40 | 25 | 5 |
| 10. | 40 | 30 | 25 | 5 |
| 11. | 45 | 45 | 10 | 0 |
| 12. | 45 | 40 | 10 | 5 |

Other than these components, other solvents known to those skilled in the art suitable for use in the present invention can be used to prepare the liquid formulation, and combinations thereof, including but not limited to alcohols such as but not limited to (methyl, ethyl, butyl, propyl, isopropyl, isopropyl myristate, etc.), glycols such as, but not limited to (propylene, polyethylene, glycerin, etc.) mineral oils, vegetable oils, and others.

### Example

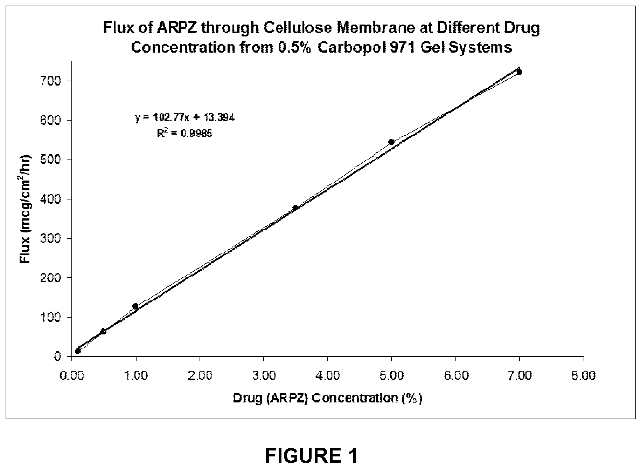

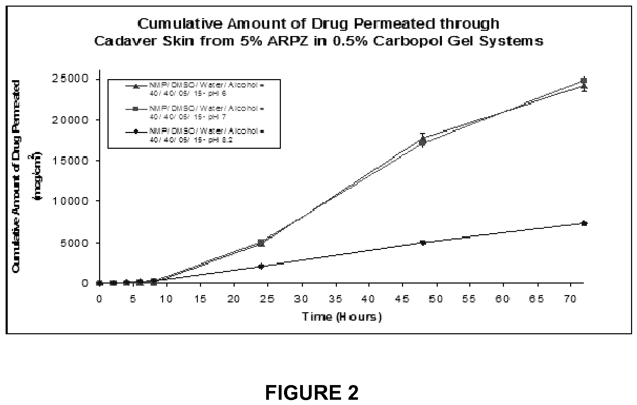

The effect of gelling agents and their concentration on the permeation of ARPZ through artificial membranes and human cadaver skin was evaluated and two characteristic graphs are shown in Figures 1 & 2. The optimal desired composition of ARPZ gel formulation contains 0.5% W/V Carbopol 971. ARPZ can be gelled by gelling agents, including but not limited to, natural polymers (such as agar, alginic acid and derivatives, cassia tora, collagen, gelatin, gellum gum, guar gum, pectin, potassium, or sodium carageenan, tragacanth, xanthan, etc), semisynthetic polymers (such as methylcellulose, carbosymethylcellulose, hydroxypropylcellulose, hydroxypropylmethylcellulose, etc.) synthetic polymers (such as carboxyvinyl polymers or carbomers: carbopol 940, carbopol 934, carbopol 971, poloxamer, polyacrylamide, polyvinyl alcohol, polyethylene, and its co-polymers etc), and clays (such as silicates, etc). In addition, other than cellulose membranes, ARPZ can be evaluated with other artificial membranes including but not limited to silicone membranes (polydimethylsiloxane), liposoem-coated membranes, solid-supported liquid membranes, lecithin organogel membranes and other. Besides the gel formulations of ARPZ, other dosage forms including, but not limited to, ointments, creams, emulsions, liposomes, etc. may be used.

Referring now to Figure 1 there is shown the Effect of Drug Concentration on the Flux of ARPZ through Cellulose Membrane from 0.5% Carbopol 971 Gel Systems.

Referring also to Figure 2 there is shown the Cumulative Amount of 5% ARPZ Permeated through Cadaver Skin from 0.5% Carbopol Gel System. The lowermost line shows an example.

### Example

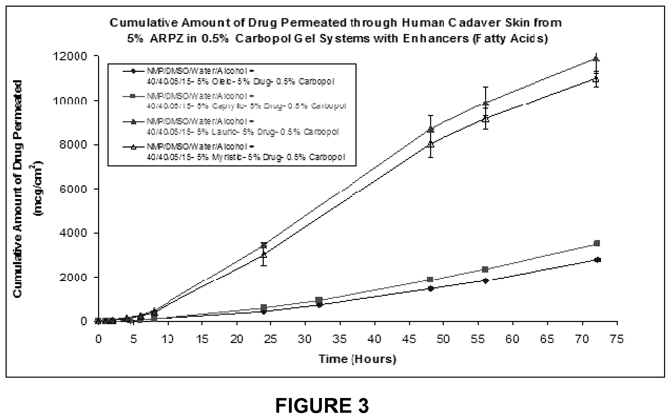

The effect of enhancers on the flux of ARPZ through human cadaver skin was evaluated and is shown in Figure 3. The desired optimum composition of ARPZ gel formulation contained Lauric and Myristc acid. Apart from Lauric and Myristc acid enhancer, the ARPZ transdermal delivery can be influenced by enhancers including but not limited to water, sulfoxides, and similar chemicals, dimethylsulfoxide (DMSO), dimethylacetamide (DMAC), dimethylformamide (DMF), decymethylsulfoxide (DCMS) etc, azone, pyrrolidones N-methyl-2-pyrrolidone (NMP), 2-pyrrolidon (2p), etc., fatty acids esters (butyl ethanoate, ethyl ethanoate, ethyl oleate, isopropyl myristate, isopropyl palmiate, methyl ethanoate etc), fatty acids (capric, caprylic, lauric, oleic, myristic, linoleic, stearic, palmitic etc), alcohols, fatty alcohols and glycols (nathanol, dodecanol, propylene glycols, glycerol etc), urea, essential oils, terpene and terpenoids (limonene, thymol, cineole etc), liposomes, niosomes, transferomes, ethanosomes etc.

Referring now to Figure 3 there is shown the Cumulative Amount of Drug Permeated through Human Cadaver Skin from 5% ARPZ in 0.5% Carbopol Gel Systems with Enhancers (Fatty Acids).

### Example

The effects of pH on the permeation of ARPZ through human cadaver skin were evaluated and a characteristic graph is shown in Figure 2. The preferred optimum composition of ARPZ gel transdermal formulation had a pH in the range of approximately 6 to 7. Other than these optimal pH values, the ARPZ transdermal delivery may be influenced by pH values outside of the preferred range, but to a lesser extent. Thus, the present invention may still be achieved outside of the preferred pH range of approximately 6 to7, depending upon the circumstances of use.
The systems of this discovery can deliver ARPZ at a flux between 50mcg/ch-2. h and 800mcg/ch-2.h, which can produce the required therapeutic ARPZ blood levels. Flux rate can be changed by modifying such parameters as ARPZ initial concentration, surface area of the patch, pH of the formulation, vehicle composition, enhancer type and composition, etc., in accordance with the teachings of the present invention.
Optimum therapeutic outcome requires not only a proper drug selection but also an effective drug delivery. Psychotropic drug compliance of rigorous regular medication schedules is of great importance. In many instances, oral administration of psychotropic agents is considered a less than optimal delivery system due to patient non-compliance⁵. Transdermal delivery of psychotropic drugs, especially with prolonged duration of action, would be valuable in increasing medication compliance, especially in the geriatric population. Further, potential advantages of ARPZ transdermal delivery are as follows: lack of hepatic first pass effect; eliminating the potential for over- or under- dosing; allowing the flexibility of terminating the drug administration by simply removing the patch; providing a simplified therapeutic regimen, thereby assisting medication compliance in the geriatric population.

### References

1. Inoue,T., Domae,M.,Yamada,K.,and Furukawa,T. Effects of the novel antipsychotic agent 7-([4-2,3-dichlorophenylo-1-piperazinyl] b Neuroutyloxyo-3,4-dihydro2 (1H) -quinolinone (OPC-14597) on prolactin release from the rat anterior pituitary. J.Pharmacol. Exp. Ther. 1996;277(1):137-143.
2. Burris,K.D., Moiski,T.F., Ryan,E., Xu,C., Tottori,K., Kikuchi,T., Yocca,F.D, and Molinoff,P.B. Aripiprazole is a high affinity partial agonist at human D2 dopamine receptors. Int. J.Neuropsychopharmacol. 2000;3(Suppl.1), S129.
3.Petrie,J.L., Saha,A.R., and McEvoy,J.P. Aripiprazole, a new atypical antipsychotic: Phase II clinical trial results. Eur.Neuropsychopharm 1997; 7 (Suppl 2): S227.
4. Saha,A.R., McQuade,R., Carson,W.H.,Ali,M.,W.,Durbar,G.C.,and Ingenito,G. Efficacy and safety of Aripiprazole and Risperidone vs.Placebo in patients with schizophrenia and schizoaffective disorder. World J. Biol Psych 2001; 2 (Suppl 1): 305S.
5. Geeta,A., Sanju, D., Psychotropic Drugs and Transdermal Delivery. An Overview. Int. J. of Pharma and Bio Science, 2001;V 1(2).

## Claims

1. A pharmaceutical composition comprising aripiprazole in a gel dosage form for transdermal delivery, wherein the composition comprises:
a) aripirazole in the amount of 1 to 20% w/v;
b) a gelling agent in the range of about 0.1% to 5% w/v selected from the group consisting of natural polymers, semisynthetic polymers, synthetic polymers, carboxyvinyl polymers or carbomers, carbopol 940, carbopol 934, carbopol 971, poloxamer, polyacrylamide, polyvinyl alcohol, polyethylene and co-polymers thereof;
c) a solvent mixture having 40% N-methyl-2-pyrrolidone, 40% Dimethylsulfoxide, 15% alcohol and 5% water, wherein the alcohol is ethyl alcohol;
d) a permeation enhancer.
and wherein the pH of the composition is 6 to 7.

2. The pharmaceutical composition of claim 1, wherein the permeation enhancer is selected from the group consisting of lauric acid, myristc acid, sulfoxides, dimethylsulfoxide, dimethylacetamide, dimethylformamide, decymethylsulfoxide, azone, pyrrolidones, fatty acid esters, fatty acids, alcohols, fatty alcohols and glycols, urea, essential oils, terpene and terpenoids, liposomes, niosomes, transferomes and ethanosomes.

3. A pharmaceutical composition comprising aripiprazole in a liquid dosage form for transdermal delivery, wherein the composition comprises:
a) aripirazole in the amount of 1 to 20% w/v;
b) a solvent mixture having 40% N-methyl-2-pyrrolidone, 40% Dimethylsulfoxide, 15% alcohol and 5% water, wherein the alcohol is ethyl alcohol;
wherein the pH of the composition is 6 to 7.

4. The pharmaceutical composition of Claim 3 being in the form of a liquid and comprising an alcohol, glycol, mineral oil, and/or vegetable oil.

5. The pharmaceutical composition of Claim 4, further comprising a permeation enhancer.

6. The pharmaceutical composition of Claim 5, wherein the permeation enhancer is selected from the group consisting of lauric acid, myristc acid, sulfoxides, dimethylsulfoxide, dimethylacetamide, dimethylformamide, decymethylsulfoxide, azone, pyrrolidones, fatty acid esters, fatty acids, alcohols, fatty alcohols and glycols, urea, essential oils, terpene and terpenoids, liposomes, niosomes, transferomes and ethanosomes.

## Patentansprüche

1. Pharmazeutische Zusammensetzung, umfassend Aripiprazol in einer Geldosierungsform zur transdermalen Verabreichung, wobei die Zusammensetzung Folgendes umfasst:
a) Aripiprazol in der Menge von 1 bis 20 % w/v;
b) ein Geliermittel im Bereich von etwa 0,1 % bis 5 % w/v, ausgewählt aus der Gruppe bestehend aus natürlichen Polymeren, halbsynthetischen Polymeren, synthetischen Polymeren, Carboxyvinylpolymeren oder Carbomeren, Carbopol 940, Carbopol 934, Carbopol 971, Poloxamer, Polyacrylamid, Polyvinylalkohol, Polyethylen und Co-Polymeren davon;
c) ein Lösungsmittelgemisch mit 40 % N-Methyl-2-pyrrolidon, 40 % Dimethylsulfoxid, 15 % Alkohol und 5 % Wasser, wobei der Alkohol Ethylalkohol ist;
d) einen Permeationsverstärker;
und wobei der pH der Zusammensetzung 6 bis 7 beträgt.

2. Pharmazeutische Zusammensetzung nach Anspruch 1, wobei der Permeationsverstärker ausgewählt ist aus der Gruppe bestehend aus Laurinsäure, Myristinsäure, Sulfoxiden, Dimethylsulfoxid, Dimethylacetamid, Dimethylformamid, Decylmethylsulfoxid, Azon, Pyrrolidonen, Fettsäureestern, Fettsäuren, Alkoholen, Fettalkoholen und Glycolen, Harnstoff, ätherischen Ölen, Terpen und Terpenoiden, Liposomen, Niosomen, Transferomen und Ethanosomen.

3. Pharmazeutische Zusammensetzung umfassend Aripiprazol in einer flüssigen Dosierungsform zur transdermalen Verabreichung, wobei die Zusammensetzung Folgendes umfasst:
a) Aripiprazol in der Menge von 1 bis 20 % w/v;
b) ein Lösungsmittelgemisch mit 40 % N-Methyl-2-pyrrolidon, 40 % Dimethylsulfoxid, 15 % Alkohol und 5 % Wasser, wobei der Alkohol Ethylalkohol ist;
wobei der pH der Zusammensetzung 6 bis 7 beträgt.

4. Pharmazeutische Zusammensetzung nach Anspruch 3, die in Form einer Flüssigkeit vorliegt und einen Alkohol, ein Glycol, Mineralöl und/oder Pflanzenöl umfasst.

5. Pharmazeutische Zusammensetzung nach Anspruch 4, ferner umfassend einen Permeationsverstärker.

6. Pharmazeutische Zusammensetzung nach Anspruch 5, wobei der Permeationsverstärker ausgewählt ist aus der Gruppe bestehend aus Laurinsäure, Myristinsäure, Sulfoxiden, Dimethylsulfoxid, Dimethylacetamid, Dimethylformamid, Decylmethylsulfoxid, Azon, Pyrrolidonen, Fettsäureestern, Fettsäuren, Alkoholen, Fettalkoholen und Glycolen, Harnstoff, ätherischen Ölen, Terpen und Terpenoiden, Liposomen, Niosomen, Transferomen und Ethanosomen.

## Revendications

1. Composition pharmaceutique comprenant de l'aripiprazole sous une forme posologique en gel pour une administration transdermique, dans laquelle la composition comprend :
a) de l'aripirazole en une quantité de 1 à 20 % p/v ;
b) un agent gélifiant dans la plage d'environ 0,1 % à 5 % p/v sélectionné parmi le groupe constitué de polymères naturels, de polymères semi-synthétiques, de polymères synthétiques, de polymères ou de carbomères de carboxyvinyle, du carbopol 940, du carbopol 934, du carbopol 971, de poloxamère, de polyacrylamide, d'alcool polyvinylique, de polyéthylène et de copolymères de ceux-ci ;
c) un mélange de solvants constitué de 40 % de N-méthyl-2-pyrrolidone, de 40 % de diméthylsulfoxyde, de 15 % d'alcool et de 5 % d'eau, dans lequel l'alcool est l'alcool éthylique ;
d) un accélérateur de perméation
et dans laquelle le pH de la composition varie de 6 à 7.

2. Composition pharmaceutique selon la revendication 1, dans laquelle l'accélérateur de perméation est choisi parmi le groupe constitué de l'acide laurique, de l'acide myristique, de sulfoxydes, de diméthylsulfoxyde, de diméthylacétamide, de diméthylformamide, de décyméthylsulfoxyde, d'azone, de pyrrolidones, d'esters d'acides gras, d'acides gras, d'alcools, d'alcools gras et de glycols, d'urée, d'huiles essentielles, de terpène et de terpénoïdes, de liposomes, de niosomes, de transféromes et d'éthanosomes.

3. Composition pharmaceutique comprenant de l'aripiprazole sous une forme posologique liquide pour une administration transdermique, dans laquelle la composition comprend :
a) de l'aripirazole en une quantité de 1 à 20 % p/v ;
b) un mélange de solvants constitué de 40 % de N-méthyl-2-pyrrolidone, de 40 % de diméthylsulfoxyde, de 15 % d'alcool et de 5 % d'eau, dans lequel l'alcool est l'alcool éthylique ;
dans laquelle le pH de la composition varie de 6 à 7.

4. Composition pharmaceutique selon la revendication 3 étant sous la forme d'un liquide et comprenant un alcool, un glycol, une huile minérale et/ou une huile végétale.

5. Composition pharmaceutique selon la revendication 4, comprenant en outre un accélérateur de perméation.

6. Composition pharmaceutique selon la revendication 5, dans laquelle l'accélérateur de perméation est choisi parmi le groupe constitué de l'acide laurique, de l'acide myristique, de sulfoxydes, de diméthylsulfoxyde, de diméthylacétamide, de diméthylformamide, de décyméthylsulfoxyde, d'azone, de pyrrolidones, d'esters d'acides gras, d'acides gras, d'alcools, d'alcools gras et de glycols, d'urée, d'huiles essentielles, de terpène et de terpénoïdes, de liposomes, de niosomes, de transféromes et d'éthanosomes.
